Europäisches Patentamt

European Patent Office

Office européen des brevets

(19)

(11) Veröffentlichungsnummer: **0 117 262**
**B1**

(12) **EUROPÄISCHE PATENTSCHRIFT**

(45) Veröffentlichungstag der Patentschrift:
**20.04.88**

(21) Anmeldenummer: **83101863.5**

(22) Anmeldetag: **25.02.83**

(51) Int. Cl.⁴: **G 01 N 33/50,** G 01 N 33/53,
G 01 N 33/552, G 01 N 33/58,
G 01 N 35/00, C 12 Q 1/00

(54) Verfahren und Vorrichtungen für Untersuchungen an unbeweglich gemachtem biologischem Material.

(43) Veröffentlichungstag der Anmeldung:
**05.09.84 Patentblatt 84/36**

(45) Bekanntmachung des Hinweises auf die Patenterteilung:
**20.04.88 Patentblatt 88/16**

(84) Benannte Vertragsstaaten:
**AT BE CH DE FR GB IT LI NL SE**

(56) Entgegenhaltungen:
DD - A - 145 133
DE - A - 3 107 964
US - A - 3 736 042

**S. RÄISÄNEN, I. RANTALA, H. HELIN "Journal of Clinical Pathology", Band 33, 1980, Seiten 95,96**

**Die Akte enthält technische Angaben, die nach dem Eingang der Anmeldung eingereicht wurden und die nicht in dieser Patentschrift enthalten sind.**

(73) Patentinhaber: **Stöcker, Winfried, Dr. med., Am Sonnenberg 9, D-2401 Gross Grönau (DE)**

(72) Erfinder: **Stöcker, Winfried, Dr. med., Am Sonnenberg 9, D-2401 Gross Grönau (DE)**

(74) Vertreter: **UEXKÜLL & STOLBERG Patentanwälte, Beselerstrasse 4, D-2000 Hamburg 52 (DE)**

ACTORUM AG

## Beschreibung

Die vorliegende Erfindung beschreibt ein rationelles Verfahren und Vorrichtungen für Untersuchungen an unbeweglich gemachtem biologischem Material.

Stand der Technik

Der Stand der Technik und die Wirkungsweise der Erfindung sollen am Beispiel der Immunfluoreszenzuntersuchung an Gefrierschnitten dargestellt werden.

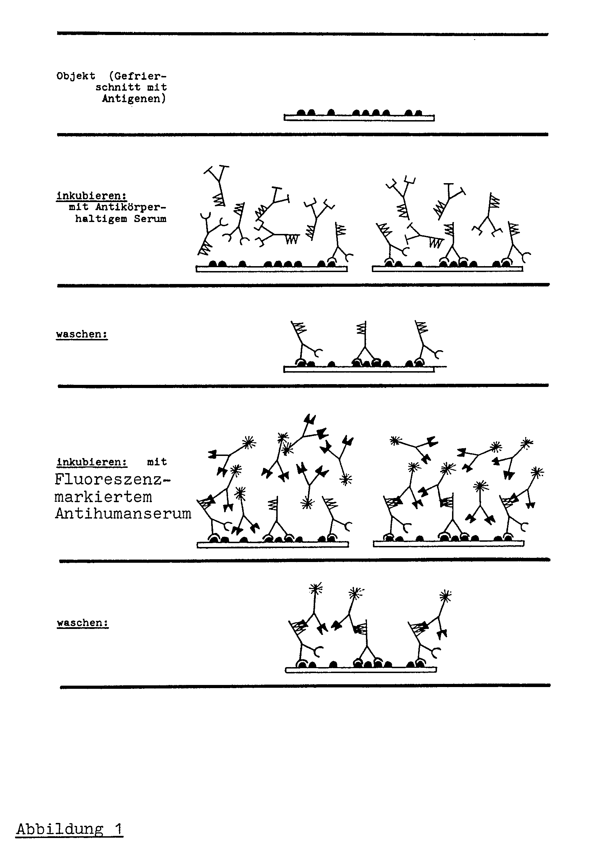

Mit dieser Untersuchung können bei manchen Kranken Antikörper gegen körpereigenes Gewebe nachgewiesen werden. Die Methode wurde von Coons und anderen eingeführt (Coons, A.H., Creech, H.J., Jones, R.N., Proc. Soc. Exp. Biol. N.Y. 47, 1941, Ss. 200 ff): Man bringt einen Gefrierschnitt gesunden Gewebes auf eine Glasoberfläche und lässt ihn auftauen und antrocknen. Wie in Abb. 1 dargestellt, wird er mit dem verdünnten Serum eines Kranken überschichtet. Enthält das Serum Antikörper gegen die Antigene des Gewebes, dann bleiben sie am Gefrierschnitt hängen. Antikörper, die nicht gegen Antigene des Gefrierschnitts gerichtet sind, binden sich nicht und werden abgewaschen.

In einem zweiten Schritt werden aus Tieren gewonnene, mit einem fluoreszierenden Stoff markierte Antikörper aufgebracht, die gegen menschliche Antikörper gerichtet sind (Fluoreszenzmarkiertes Antihumanserum). Sie binden sich an ggf. am Gefrierschnitt hängende menschliche Antikörper und können nicht abgewaschen werden.

Enthielt das Serum des Kranken Antikörper gegen das untersuchte Gewebe, dann lässt sich jetzt im Fluoreszenzmikroskop der an die entsprechenden Gewebestrukturen gebundene Fluoreszenzfarbstoff nachweisen.

Häufig wird auch Gewebe von Kranken untersucht, um festzustellen, ob sich in vivo an bestimmte Strukturen Antikörper gebunden haben. Dazu werden Gefrierschnitte von diesem Gewebe angefertigt und in einem direkten Immunfluoreszenztest unmittelbar mit einem Fluoreszenzmarkierten Antihumanserum zusammengebracht, der erste Schritt des oben beschriebenen indirekten Immunfluoreszenztests entfällt. Die Antikörper enthaltenden Strukturen fluoreszieren im gewaschenen Präparat.

Es ist üblich, die Gefrierschnitte auf Standardobjektträger aus Glas zu montieren, und zwar wird in der Regel für jeden Gefrierschnitt ein Standardobjektträger genommen. Eine eingehende Beschreibung des Tests findet sich z.B. bei Storch (Storch, W.: «Immunfluoreszenzfibel», Fischer-Verlag Jena, 1979). Der Test birgt einige Fehlerquellen, setzt viel Übung voraus und verlangt einen grossen Arbeits- und Materialaufwand.

Oft kommt es vor, dass die Tropfen verlaufen und die Gefrierschnitte dann antrocknen (wenn die Gefrierschnitte während des Tests trocken werden, lassen sich die Ergebnisse meistens nicht mehr verwerten). Vorsichtshalber macht man daher grosse Tropfen und vergeudet Reagenzien. Vor dem Mikroskopieren überschichtet man den Gefrierschnitt mit $p_H$-gepuffertem Glycerin und legt ein Deckglas darüber. Das Deckglas sollte auf dem Glycerin schwimmen. Wird zuviel Glycerin aufgetropft, dann verrutscht das Deckglas und das Mikroskop wird verunreinigt. Deshalb tupft man überschüssiges Glycerin ab. Manchmal entfernt man dabei zuviel Glycerin: Dann wird durch Kapillarkraft das Deckglas fest an den Objektträger gezogen und zerquetscht den Gefrierschnitt, oder man verschiebt das Deckglas versehentlich gegen den Standardobjektträger und zerstört den Gefrierschnitt dabei.

Das gleiche passiert, wenn man beim Mikroskopieren den Gefrierschnitt in die Brennebene des Objektivs stellen will und dabei mit dem Deckglas zu nahe an das Objektiv kommt. Ein positives Ergebnis kann dadurch scheinbar negativ werden.

Die Seren verschiedener Kranker können gleichzeitig auf einem Objektträger untersucht werden. Dadurch werden Arbeitsabläufe zusammengefasst, und das Testverfahren wird einfacher. Man muss zuvor mehrere Gefrierschnitte nebeneinander aufbringen und darauf achten, dass sich die Seren untereinander nicht vermischen. Das wird erleichtert durch eine Aufteilung in Reaktionsfelder 3, die voneinander durch eine wasserabstossende Beschichtung 4 des Objektträgers abgesetzt sind (O'Neill, P., Johnson, G.D.; Ann. N.Y. Acad. Sci. 177, 446–452, 1971; US-A-3 736 042; EP-A-18 435 oder durch Farbringe (Räisänen, S. und andere, J. clin. Pathol. 33, 95–96, 1980). Je mehr Seren auf einem Objektträger untersucht werden, umso geringer wird für jede Einzeluntersuchung der präparatorische Aufwand während des Tests. Mit der oben beschriebenen Technik können aber nicht mehr als zwanzig Einzeluntersuchungen nebeneinander auf demselben Objektträger durchgeführt werden, vor allem weil die Reaktionsfelder nacheinander betropft werden und sich dann unterschiedliche Inkubationszeiten für die einzelnen Analysen ergeben und weil einige Präparate austrocknen und unbrauchbar werden, während die anderen mit dem Fluoreszenz-markierten Antihumanserum betropft werden.

Für die Untersuchung sehr vieler Seren nebeneinander, beispielsweise 96 auf einem Objektträger (Platte Abb. 2), bieten sich die Methoden nach Stöcker (EP-A-18 435; DE-A-3 107 964) an: Auf zwei Platten befinden sich in kongruenter Anordnung hydrophile Reaktionsfelder, die von einer wasserabstossenden Schicht umgeben sind.

Auf die Reaktionsfelder der einen Platte werden Gefrierschnitte montiert, auf die Reaktionsfelder der anderen Platte tropft man Proben, z.B. Serumverdünnungen oder das Fluoreszenz-markierte Antihumanserum. Beide Platten werden dann in ein Gestell gelegt, und zwar so, dass die Gefrierschnitte in die flüssigen Proben eintauchen. Alle Gefrierschnitte einer Platte inkubieren gleich lange, und kein Gefrierschnitt trocknet während des Tests an, auch nicht beim Aufbringen des Fluoreszenz-markierten Antihumanserums.

Die rationelle Methode zur Untersuchung vieler Seren auf einer Platte hat sich bisher in der Im-

munfluoreszenzdiagnostik nur dort bewährt, wo suspendierfähige Antigene verwendet werden können, weil man sie auf die Reaktionsfelder tropfen kann, z.B. die Erreger der Lues oder der Toxoplasmose. Wer mit der Technik des Gefrierschneidens vertraut ist, weiss, wie mühsam es ist, mit den bisher bekannten Verfahren 96 Gefrierschnitte sauber und gleichmässig auf die Reaktionsfelder einer Platte zu montieren. Bei einer industriellen Fertigung müsste man mit einer hohen Ausschussziffer rechnen.

Häufig ist es nötig, in einem Serum nach Antikörpern gegen verschiedene Antigene zu suchen: Mehrere suspendierfähige Antigene können dazu nebeneinander auf ein Reaktionsfeld getropft werden. Nach dem Antrocknen und ggf. Fixieren werden sie gemeinsam mit einem Tropfen Serum-Verdünnung überschichtet (Wang, S.P., Excerpta Medica, Amsterdam, 273–288, 1971). Will man Antikörper gegen verschiedene Gewebe nachweisen, stellt man mehrere Gefrierschnitte zu einem «Bunten Schnitt» zusammen, den man mit einem grossen Tropfen Probe oder Reagenz überschichtet. Man kann dazu die Gefrierschnitte für jedes Gewebe einzeln herstellen und unterschiedliche Gefrierschnitte nebeneinander auf ein Reaktionsfeld legen. Oder man friert mehrere Stückchen verschiedener Gewebe in einer wässrigen Lösung von Carboxymethylcellulose gemeinsam nebeneinander ein und schneidet und montiert sie gleichzeitig (z.B. Nairn, R.C.: «Fluorescent Protein Tracing», Churchill Livingstone Edinburgh, 1976). Man kann nur wenige Organstückchen zusammen in einem Block schneiden, und die Organstückchen müssen genau «zurechtgetrimmt» werden. Dazu ist viel Geschick nötig und es geht Gewebe dabei verloren. Unterschiedlich zu fixierende Gefrierschnitte können nicht im selben «Bunten Schnitt» nebeneinander liegen.

Manchmal steht nur sehr wenig zu untersuchendes Serum oder Fluoreszenz-markiertes Antihumanserum zur Verfügung. Dann versucht man, den Testansatz so klein wie möglich zu halten. Voraussetzung dafür sind kleine Gefrierschnitte. Man schneidet das Gewebe auf die gewünschte Grösse zurecht und nimmt dabei in Kauf, dass viel Material verlorengeht.

Untersucht man ungleichmässig im Gewebe verteilte Strukturen, z.B. Langerhans'sche Inseln der Bauchspeicheldrüse oder Glomeruli der Niere, dann muss man grosse Schnitte anfertigen um möglichst sicher zu sein, dass man die gewünschten Strukturen beim Mikroskopieren vorfindet. Es wäre zu schwierig, einzelne Inseln oder Glomeruli aus dem losen Gefrierschnitt herauszuschneiden.

Manchmal sind die zur Verfügung stehenden Organstückchen so klein, dass man nur sehr wenige Gefrierschnitte daraus gewinnen kann, die für die verschiedenen durchzuführenden Untersuchungen nicht ausreichen. Die interessierenden Strukturen sind aber auf diesen Schnitten oft mehrfach vertreten. Man kann dann versuchen, die fertigen losen Gefrierschnitte frisch oder nach Gefriertrocknung zu teilen, die gewünschten Strukturen sind aber am losen Schnitt kaum zu erkennen und die Manipulation ist sehr schwierig.

Eine besondere Schwierigkeit bei biochemischen Untersuchungen an Gefrierschnitten besteht darin, dass sie während der Inkubationen häufig nur schlecht auf der Unterlage haften. Oft schwimmen sie ganz oder teilweise ab.

Werden Glasobjektträger verwendet, müssen sie daher vor dem Aufbringen der Gefrierschnitte sorgfältig gereinigt werden (Chromschwefelsäure, Ethanol, Aceton; Storch, W., siehe oben). In einigen Labors überzieht man die Glasoberfläche mit Glycerin und Gelatine oder mit Hühnereiweiss (Romeis, B.: «Mikroskopische Technik», Oldenbourg-Verlag München, Wien, 1968). Dieses Verfahren ist keine wesentliche Verbesserung und wird von vielen Untersuchern abgelehnt. Für einige Gewebe, wie Lunge, Darmschleimhaut und besonders für fettreiche Gewebe (z.B. Pankreas, Nebennierenmark) gibt es bisher kein zuverlässiges Verfahren, unifixierte Gefrierschnitte fest auf ihrer Unterlage zu halten, besonders wenn die Inkubationen lange dauern und wenn gründlich gewaschen werden muss. Man kann einen Gefrierschnitt bei −30°C kurz in Methylenchlorid oder in ein anderes organisches Lösungsmittel tauchen und das Fett herauslösen, wodurch die Haftung verbessert wird (Stöcker, W., nicht publ.). Dabei werden aber in einigen Fällen Antigene oder Enzyme zerstört, ebenso durch sonst in der Histochemie gebräuchliche Fixierverfahren. In der Immunhistochemie verwendet man daher vorwiegend unfixiertes Gewebe (Wick, G. und andere: «Immunfluorescence», Medizinische Verlagsgesellschaft Marburg/Lahn, 1978). Nur wenn das Antigen in Wasser löslich ist, wird der Gefrierschnitt fixiert, z.B. bei der Schilddrüse, deren Kolloid durch Behandlung mit absolutem Methanol unlöslich wird. Es sind bereits sehr viele Verfahren bekannt, mit denen organisches Material an aktivierte Oberflächen gebunden werden kann. Insbesondere bemüht man sich darum, Enzyme, Antigene und Antikörper an Festkörpern zu immobilisieren (z.B. Ternynck, T., Avrameas, S., FEBS-Letters 23, 24–28, 1972; Guesdon, L.D. und andere, J. Immunol. Meth. 21, 59–63, 1978; DE-A-2 102 514; DE-A-2 740 008; DE-A-2 749 317; DE-C-2 905 657). Es wurde auch schon beschrieben, in Polyacrylamid eingebettetes Gewebe zu schneiden und die Schnitte chemisch an eine mit reaktiven Gruppen versehene Oberfläche zu binden (Hausen, P., Dreyer, C., Stain Technol. 56, 287–293, 1981). Dabei ging man davon aus, dass sich das Polyacrylamid bindet und nicht das Gewebe. Dass sich ein Gefrierschnitt nicht eingebetteten Gewebes, der auf einer unvorbehandelten Oberfläche nicht ausreichend haftet, über reaktive Gruppen der aktivierten Oberfläche fest mit ihr verbinden kann, wurde von den Autoren offenbar nicht erkannt, und es gibt in der Fachliteratur keine Berichte darüber.

Aufgabe der Erfindung und Beschreibung

Aufgabe der Erfindung ist es, ein verbessertes Verfahren herauszufinden, mit dem Untersuchungen an unbeweglich gemachtem biologischem Material durchgeführt werden können, und Vorrichtungen dafür zu schaffen.

Die Erfindung löst diese Aufgabe. Mit Hilfe der Erfindung gelingt es, biologisches Material an eine Oberfläche zu binden und es mit biochemischen Methoden rationell zu untersuchen oder es für rationelle biochemische Untersuchungen einzusetzen. Bei der Beschreibung hält sich der Erfinder an das Beispiel der Immunfluoreszenz an Gefrierschnitten biologischen Gewebes. Die Erfindung eignet sich aber auch für viele Anwendungen aus anderen Bereichen der Immunologie (Fluoreszenzimmunoassay, Radioimmunoassay, Enzymimmunoassay), der Histochemie, der Mikrobiologie, der klinischen Chemie und der Hormonchemie.

Die Erfindung umfasst ein Verfahren für Untersuchungen an unbeweglich gemachtem biologischem Material mit allgemein-biochemischen und mit histochemischen Methoden, insbesondere Methoden der Enzym-, Immun- und Hormonchemie, bei dem eine oder mehrere Untersuchungen nebeneinander auf einer Platte (2) ablaufen und bei dem das biologische Material gegen eine Beschädigung weitgehend geschützt ist, wobei das biologische Material zunächst auf der Oberfläche eines oder mehrerer Träger (1) zur Haftung gebracht wird und danach die Träger oder Teile (1a) der Träger zusammen mit dem biologischen Material auf der Platte (2) befestigt werden, wobei die das biologische Material tragende Oberfläche so angeordnet wird, dass es keinen direkten Kontakt mit festen Gegenständen bekommt und dass es während der Untersuchung nicht austrocknet, wo das unerwünscht ist.

Die Erfindung umfasst auch eine Vorrichtung zur Durchführung des Verfahrens, gekennzeichnet durch eine Platte (2) mit einem oder mit mehreren Reaktionsfeldern (3) zur Aufnahme der Träger (1) oder der Teile (1a) der Träger, in der die das biologische Material tragende Oberfläche gegen Beschädigung weitgehend geschützt ist, wobei

A. die oberste Ebene der Platte oder die oberste Ebene mit der Platte verbundener Mittel das biologische Material um den Abstand $\Delta h > 0$ überragen, oder

B. die Reaktionsfelder der Platte rundum von Bereichen der Platte umgeben sind, die die Träger oder die Teile der Träger um den Abstand $\Delta h > 0$ überragen oder

C. die Platte als Boden einer Durchflussküvette (14) ausgebildet ist, auf der ein oder mehrere Träger oder Teile der Träger befestigt sind, und deren das biologische Material tragende Oberfläche einen Abstand $\Delta h > 0$ zu einer gegenüberliegenden Wand aufweist, oder

D. durch andere geometrische (13a, 13b) oder durch mechanische oder andere Mittel ein Abstand $\Delta h > 0$ des biologischen Materials zu einer gegenüberliegenden Oberfläche einstellbar ist, und durch Träger (1) oder Teile (1a) der Träger, auf die das biologische Material zur Haftung bringbar ist, und die auf der Platte (2) befestigbar sind, wobei

a. die Träger aus nicht aktiviertem Glas, Kunststoff oder anderem Material bestehen, oder

b. die Träger aus Glas, Kunststoff oder anderem Material bestehen, und an deren Oberfläche vor dem Aufbringen des biologischen Materials reaktive Gruppen gekoppelt sind, die das biologische Material chemisch binden und

c. die Oberfläche der Träger nicht mit biologischem Material vorbeschichtet ist, oder

d. die Oberfläche der Träger mit biologischem Material vorbeschichtet ist und

e. auf die Oberfläche der Träger nicht-histologisches biologisches Material aufgebracht ist, oder

f. auf die Oberfläche der Träger histologische Dünnschnitte nicht gefrorenen biologischen Gewebes aufgebracht sind oder

g. auf die Oberfläche der Träger histologische Dünnschnitte gefrorenen biologischen Gewebes aufgebracht sind.

Die Erfindung wird im folgenden anhand mehrerer Zeichnungen näher erläutert. Es zeigen:

Abb. 1: eine schematische Darstellung des indirekten Immunfluoreszenztests,

Abb. 2: eine ebene Platte mit 96 hydrophilen Reaktionsfeldern 3, deren Umgebung 4 hydrophob beschichtet ist,

Abb. 3: die rationelle Herstellung einer Vielzahl von Gefrierschnitt-Fragmenten 1a mit Hilfe eines teilbaren Trägers 1,

Abb. 4: eine Platte 2 mit auf ihr befestigtem Träger 1a, in perspektivischer Draufsicht (a), im Querschnitt (b) und mit Deckglas im Querschnitt (c),

Abb. 5: eine schematische Darstellung, wie ein Gefrierschnitt chemisch an einen Träger gekoppelt werden kann,

Abb. 6: eine Platte 2 (a) mit auf ihr befestigten Trägern 1a und einem dazugehörenden Reagenzträger 7 (b) mit hydrophilen Reaktionsfeldern 3 und hydrophober Umgebung 4,

Abb. 7: eine schematische Darstellung des indirekten Immunfluoreszenztests gemäss der Erfindung.,

Abb. 8: eine Platte 2 (a) mit 48 auf ihr befestigten Trägern 1a, ihre Rückseite (b), den dazugehörenden Reagenzträger 7 (c) und beide während einer Inkubation im Querschnitt (d),

Abb. 9: eine Platte 2 (a) mit 1 auf ihr befestigten Träger 1a, ihre Rückseite (b), den dazugehörenden Reagenzträger 7 (c) und beide während einer Inkubation im Querschnitt (d),

Abb. 10: eine Platte 2 mit mehreren verschiedene Gefrierschnitte aufweisenden Trägern 1a auf einem Reaktionsfeld 3 («Bunter Schnitt»),

Abb. 11: eine Platte 2 (a) mit 4 «Bunten Schnitten», ihre Rückseite (b), den dazugehörenden Reagenzträger 7 (c) und beide während einer Inkubation im Querschnitt (d),

Abb. 12: eine Durchflussküvette, deren Boden durch eine Platte 2 gebildet wird, auf der mehrere Träger 1a mit Gefrierschnitten befestigt sind (ebenfalls ein «Bunter Schnitt»),

Abb. 13: eine Platte 2 mit 5 auf ihr befestigten Trägern 1a und dazu gehörendem Reagenzträger 7 während einer Inkubation, im Querschnitt (a), dieselbe Platte mit Deckglas 8 (b),

Abb. 14: eine scheibchenförmige «Platte» 2 (a), ihre Rückseite mit Griffknopf (b), den dazugehörenden Reagenzträger 7 (c), beide während einer Inkubation im Querschnitt (d) und, die «Platte» eingedeckt mit Glycerin und durch Kapillarkraft an einem Deckglas gehalten, unter dem Mikroskop.

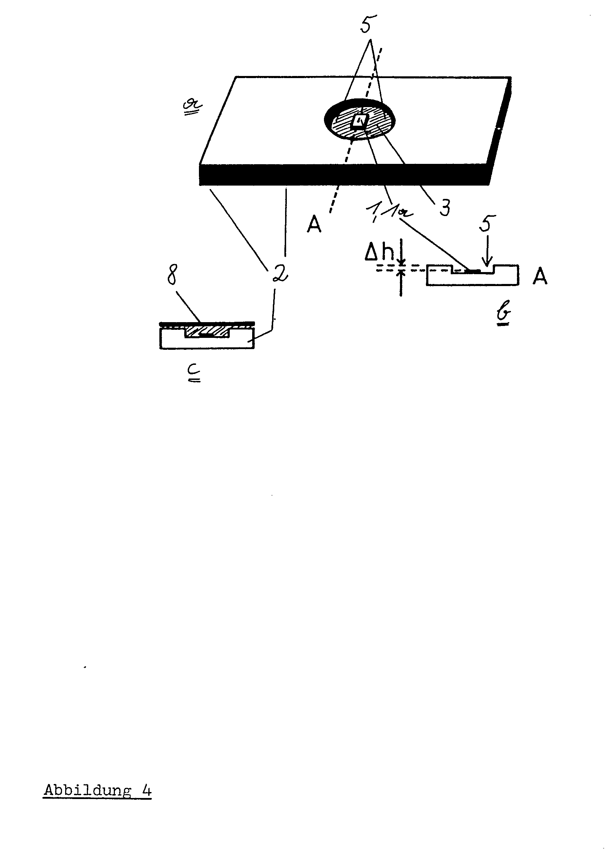

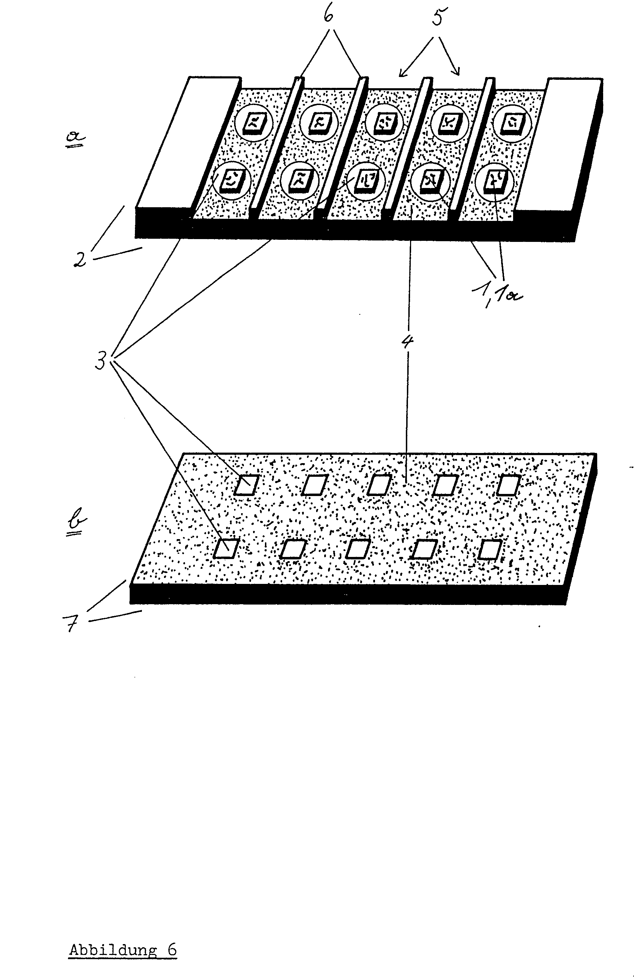

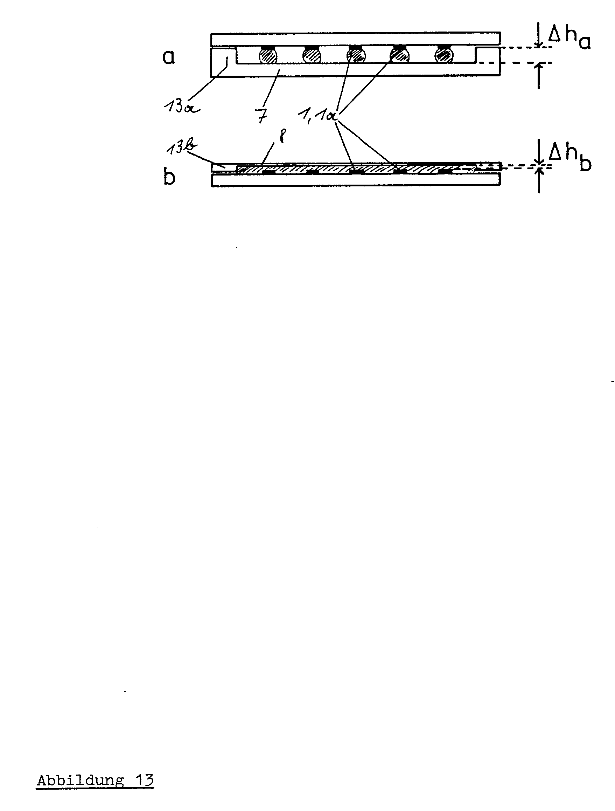

Das biologische Material wird zunächst auf die Oberfläche eines Trägers 1 gebracht, auf dem man es anhaften lässt. Ein oder mehrere Träger oder Teile 1a der Träger werden dann auf eine Platte 2 geklebt oder mit anderen Mitteln auf ihr befestigt. Auf einer planen Platte wäre jetzt, ohne dass weitere Vorkehrungen getroffen würden, das biologische Material besonders gegen Beschädigungen oder gegen Austrocknen empfindlich, weil es durch Träger und Klebeschicht über das Niveau der Platte gehoben würde. Immunfluoroeszenztests liessen sich nach der herkömmlichen Methode so nicht durchführen: Das zum Schluss aufzulegende Deckglas läge direkt auf den exponierten empfindlichen Gefrierschnitten und zerquetschte sie. Die Träger werden aber auf der Platte so angeordnet, dass das biologische Material gegen eine Beschädigung weitgehend geschützt ist und insbesondere während der Untersuchung keinen direkten Kontakt mit festem Material bekommt, und dass es während oder nach der Untersuchung nicht trocknet, wo es unerwünscht ist. Erfindungsgemäss werden die Träger zu diesem Zweck beispielsweise in Vertiefungen 5 der Platte 2 befestigt (Abb. 4, 6). Dadurch wird für das biologische Material zur obersten Plattenebene ein sicherer Abstand $\Delta h > 0$ gewährleistet. Anstelle durch ein Versenken der Träger kann auch durch andere geometrische, mechanische oder andere Mittel (Abb. 13) ein Abstand $\Delta h > 0$ des biologischen Materials zu einer gegenüberliegenden Oberfläche eingestellt werden, z.B. zu einem Deckglas 8. Wenn das biologische Material mit dem Mikroskop oder mit einem anderen Hilfsmittel beurteilt werden soll, muss dieser Abstand $\Delta h$ so festgelegt werden, dass das biologische Material in die Brennebene des Objektivs gestellt werden kann oder in eine Ebene, die der Messfühler des verwendeten Hilfsmittels erreichen kann.

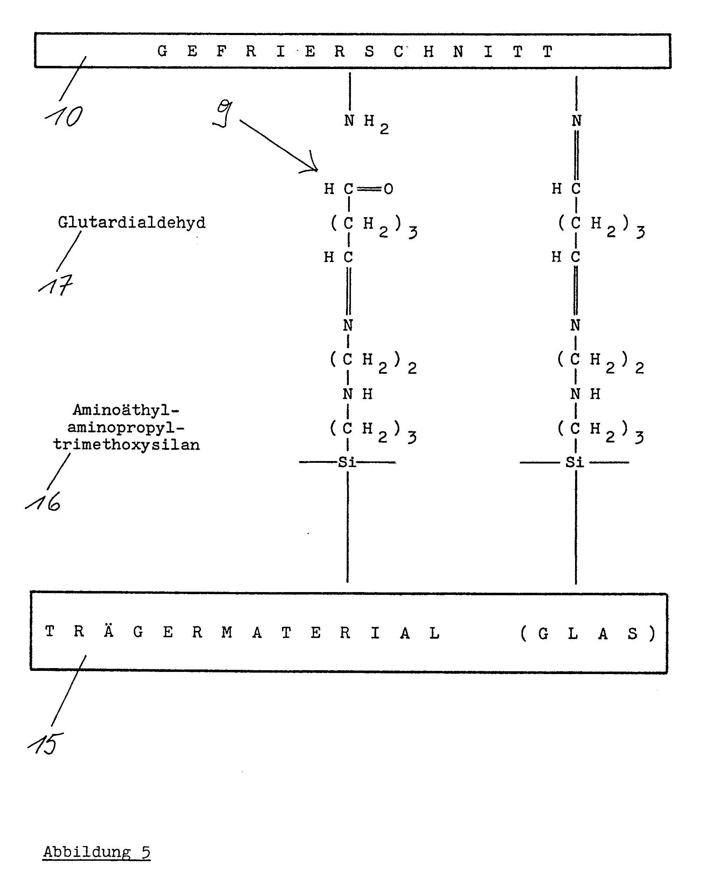

Gefrierschnitte können gemäss der Erfindung chemisch an den Träger gebunden werden, dergestalt, dass an die Oberfläche des Trägers vor dem Aufbringen der Gefrierschnitte reaktionsfähige chemische Gruppen 9 gekoppelt werden (Abb. 5). Für die Erfindung können aber auch in herkömmlicher Weise hergestellte Gefrierschnitte benutzt werden, die nicht chemisch an Träger gebunden wurden.

Mit der Erfindung werden mehrere vorteilhafte Wirkungen erzielt: Durch die das biologische Material schützende Anordnung werden die Testresultate abgesichert. Die Ergebnisse sind reproduzierbarer, der Anteil nicht gelungener Tests ist von 5–10% auf nahezu 0% abgesunken. Die Gefrierschnitte werden beim Transport und während der Tests nicht mehr so leicht beschädigt. Sie können auch von Ungeübten mit Glycerin eingedeckt werden, das Deckglas kann sie nicht mehr zerstören, auch dann nicht, wenn man fest darauf drückt.

Bei der in Abb. 4 dargestellten Anordnung liegt das Reaktionsfeld 3 in einer tieferen Ebene (Vertiefung 5) als die dem Reaktionsfeld unmittelbar benachbarte Umgebung, und auch die Oberfläche des Trägers 1a ragt nicht über die Ebene der Umgebung hinaus. Dadurch wird das Verlaufen der Serumverdünnung, des Fluoreszenz-markierten Antihumanserums oder des Glycerins verhindert, und die Gefrierschnitte trocknen nicht aus. Histologische Präparate (Dünnschnitte biologischer Gewebe), insbesondere Gefrierschnitte, oder Präparate aus anderen an Oberflächen gebundenen biologischen Materialien können mit geringem Aufwand in grosser Zahl und in guter Qualität hergestellt werden: Es ist manchmal besser, das biologische Material nicht direkt auf eine Platte zu bringen, sondern erst einen Träger damit zu beschichten und es in einem davon unabhängigen zweiten Schritt zusammen mit dem Träger auf einer Platte zu befestigen (Platte bei histochemischen Untersuchungen = Objektivträger). Man kann z.B. in einem kleinen Gefäss mit 250 ml Inhalt gleichzeitig zehntausende Glasstückchen (3 mm × 3 mm × 0,2 mm) beschichten. Die Glasstückchen können dann schnell und einfach auf eine Platte geklebt werden, ggf. durch einen Automaten. Oder man breitet das biologische Material auf einem Träger aus, lässt es sich mit dem Träger verbinden, zerteilt den Träger und klebt dann die Fragmente auf.

Die beschichteten Träger nehmen wenig Platz in Anspruch und werden bei tiefen Temperaturen nicht zerstört. Sie eignen sich daher besser für eine Tiefkühlkonservierung als zum Beispiel Standardobjektträger aus Glas. Man kann Gefrierschnitt-Fragmente (ca. 2 mm × 2 mm × 0,2 mm; als Träger z.B. Deckglasmaterial) in PVC-Schläuche oder in Glasampullen füllen, diese zuschweissen und in flüssigem Stickstoff bei −196 °C jahrelang aufbewahren, z.B. 1000 Stück in einer 1 ml fassenden Glasampulle.

Gefrierschnitte können erfindungsgemäss leicht fragmentiert werden, weil sie auf einem Träger haften, der teilbar ist. Es ist ein Kunststück, aus einem losen Gefrierschnitt einen interessierenden Bezirk passender Grösse herauszutrennen. Wird der Gefrierschnitt durch einen Träger stabilisiert, geht das sehr leicht. Für Mikroanalysen kann man aus einem 5 mm × 5 mm grossen Gefrierschnitt, der auf einem Deckglas haftet, innerhalb fünf Minuten 100 Miniatur-Gefrierschnitte herstellen. Gefrierschnitte einzelner Glomeruli können leicht aus einem auf einem Deckglas haftenden Gefrierschnitt der Niere isoliert werden. Das Gewebe wird besser ausgenutzt, weil nicht unnötig viele Glomeruli für jeden einzelnen Test verbraucht werden. Für Anteile der Gefrierschnitte, die keine Glomeruli enthalten, werden keine Reagenzien vergeudet. Die Erfindung eignet sich deshalb auch besonders gut für Untersuchungen, bei denen die Reagenzien sehr teuer sind, wie monoklonale Antikörper, oder bei denen wenig Probenmaterial zur Verfügung steht, z.B. beim Heraussuchen für die Produktion monoklonaler Antikörper geeigneter Plasmocytom-Zellen.

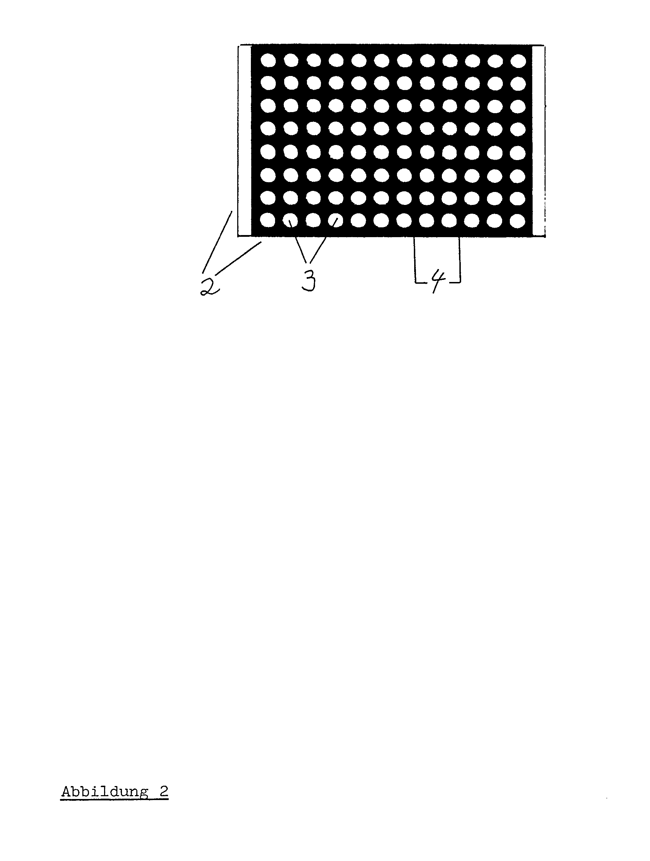

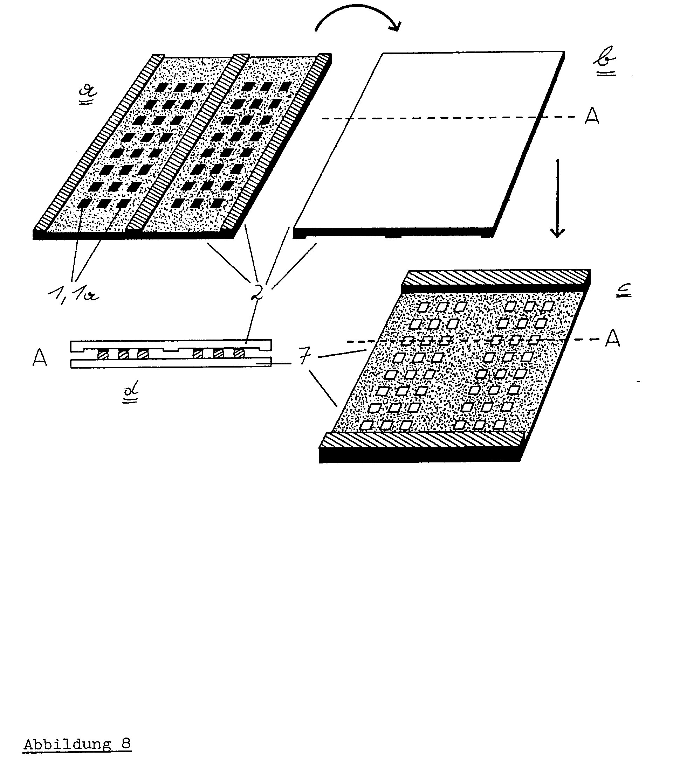

Im Gegensatz zu den bisher bekannten Verfahren lassen sich jetzt viele Gefrierschnitte leicht auf einer Platte mit vielen Reaktionsfeldern befestigen (Abb. 2, 8). Der Erfinder verwendet für Versuche Platten mit 96 Reaktionsfeldern, die jeweils einen Gefrierschnitt aufweisen.

Ebenso einfach ist es, «Bunte Schnitte» herzustellen. Die Zusammensetzung der «Bunten Schnitte» kann von Test zu Test beliebig variiert werden. Man kann Gewebe gleichzeitig nebeneinander untersuchen, die unterschiedlich vorbehandelt werden müssen, z.B. fixierte und unfixierte Gefrierschnitte der Schilddrüse: Die Gefrierschnitte werden auf dem Träger fixiert, bevor dieser fragmentiert und auf die Platte geklebt wird. Es können daher auch Fixierverfahren angewendet werden, die die hydrophobe Beschichtung der Platte angreifen.

Durch die chemische Aktivierung der Trägeroberfläche haften die Gefrierschnitte überraschend fest, und es kommt nicht mehr vor, dass sie während der Inkubationen oder während der Waschvorgänge abschwimmen, nicht einmal dann, wenn man sie mehrere Tage lang in der Flüssigkeit hält oder wenn man sie lange und kräftig überspült. Auch gefriergetrocknete lose Schnitte binden sich in wässrigem Milieu an den aktivierten Träger. Für Miniaturanalysen der Erfindung war es notwendig, die Haftung der Gefrierschnitte zu verbessern, weil sich die Gefrierschnitte von kleineren Trägerfragmenten häufig ablösten. Beispiele sollen die Wirkungsweise der Erfindung darstellen:

Beispiel 1(Abb. 5): Herstellung aktivierter Träger aus Glas

Eine ausreichende Anzahl Deckgläser, Stärke 0,2 mm, wurde 3 Tage lang in Chromschwefelsäure gereinigt und danach ausreichend lange mit destilliertem Wasser gespült. Dann wurden die Deckgläser 3 Stunden lang bei Zimmertemperatur in eine 2%ige Lösung Aminoäthylaminopropyltrimethoxysilan in Ethanol/Wasser 1/1 (Vol.) getaucht, 3 mal 1 Minute lang in absolutem Ethanol gewaschen und mit Pressluft getrocknet. Über Nacht Querpolymerisation bei 70°C, dann 3 Stunden bei Zimmertemperatur eintauchen in eine 5%ige wässrige Lösung frisch filtrierten Glutardialdehyds. Ausreichend in dest. Wasser spülen, trocknen. Die Oberfläche der Deckgläser wies jetzt selbstreagierende Aldehydgruppen 9 auf, an die sich Proteine der Gefrierschnitte über freie Aminogruppen kovalent binden konnten. Die Deckgläser wurden 6 Monate lang bei Zimmertemperatur aufbewahrt, ohne dass sie ihre Aktivität merkbar verloren.

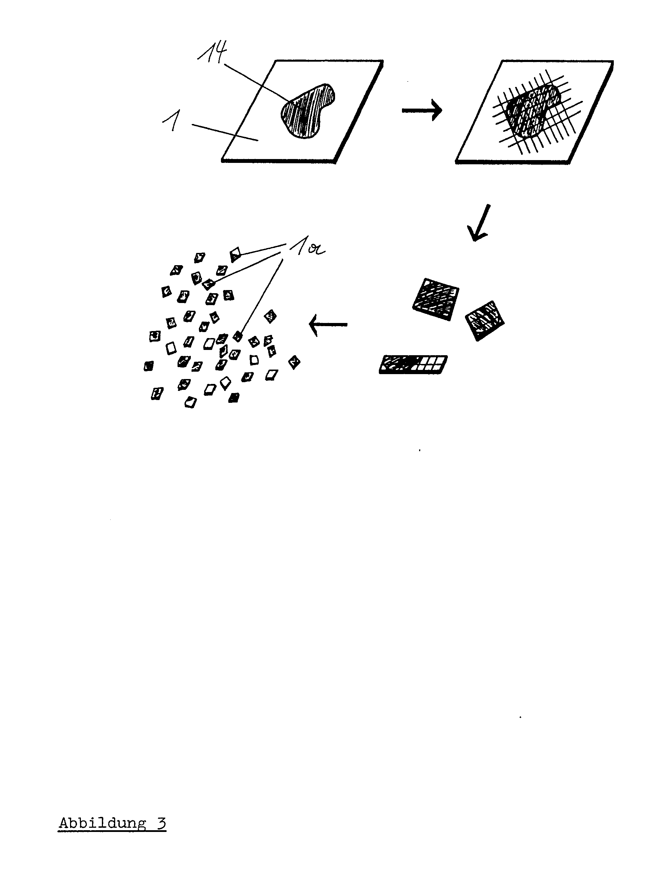

Beispiel 2 (Abb. 3): Herstellung von Gefrierschnitt-Fragmenten

Frisch hergestellte, 5 µm starke Gefrierschnitte 14 menschlicher Leber wurden auf ein nach Beispiel 1 hergestelltes Deckglas 1 gebracht, man konnte die ganze Oberfläche einer Seite des Deckglases beschichten. Man liess die Gefrierschnitte auftauen und antrocknen. Im Bereich der Gefrierschnitte wurden in das Deckglas mit einer Diamantspitze Felder eingeritzt, dann wurde es gebrochen. Ein Teil der Gefrierschnitt-Fragmente 1a wurde für das Beispiel 3 beiseite gelegt, der Rest in Schläuche aus Polyvinylchlorid verteilt, die Schläuche wurden zugeschweisst und in flüssigem Stickstoff aufgehoben.

Es ist einfacher und geht mehrfach schneller, auf diese Weise aus einem grossen Gefrierschnitt 50 Präparate herzustellen (Zeitaufwand 1/4 Minute) und dann auf eine Platte zu kleben (5 Minuten), als mit dem Gefrierschnitt-Mikrotom 50 Gefrierschnitte von einem kleinen Gewebestück zu schneiden und auf Standardobjektträger zu bringen (50 Minuten). Das Gewebe wird auch besser ausgenutzt, weil das beim Präparieren eines kleinen Gewebestückchens verlorengehende Material erhalten bleibt.

Mit Hilfe eines Lupenmikroskops können interessierende Gewebebereiche herausgesucht werden, unwichtige Bereiche und Bereiche schlechter Schnittqualität werden weggeworfen.

Das Deckglas kann schon vor dem Aufbringen der Gefrierschnitte geritzt werden. Man kann einen Gefrierschnitt auch auf eng nebeneinanderliegende Glasfragmente legen, ihn auftauen und trocknen lassen und die Fragmente dann auf einer Platte befestigen. Als Träger eignen sich auch Kunststoffe, z.B. Folien aus mit reaktiven Gruppen versehenem Polymethylmethacrylat. Aus den Folien können Fragmente gestanzt oder geschnitten werden, ggf. mit Hilfe eines LASER.

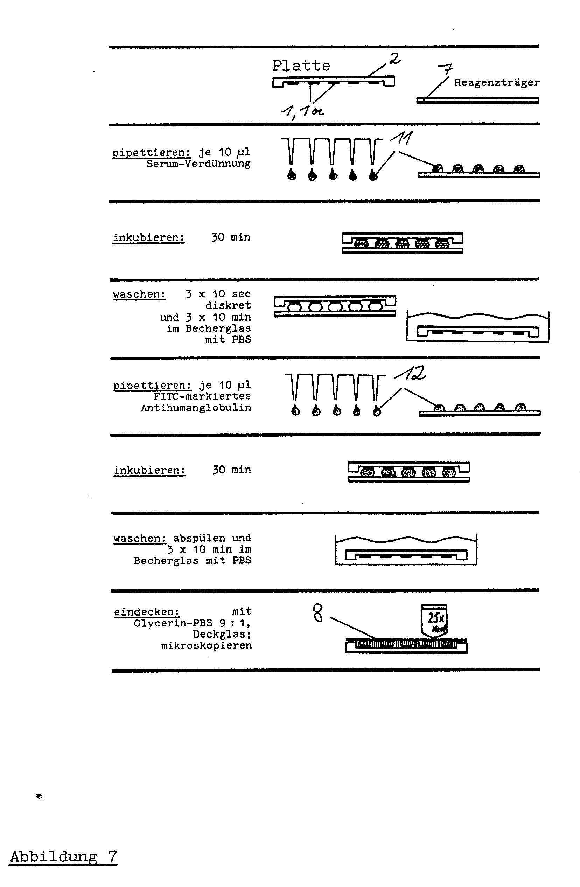

Beispiel 3 (Abb. 6 und 7): Durchführung eines Immunfluoreszenztests

Im nachfolgenden Test wurden die Seren Kranker auf Antikörper gegen Zellkerne untersucht. Dazu wurden die im Beispiel 2 hergestellten Gefrierschnitt-Fragmente 1a auf Reaktionsfelder 3a einer Platte 2 geklebt.

Die Gefrierschnitte lagen in einem um 0,1 mm tieferen Niveau als die Oberfläche der auf der Platte 2 vorhandenen Leisten 6 (Stärke 0,3 mm). Verdünnungen 11 der Seren wurden auf einen Reagenzträger 7 getropft. Die auf den Trägern 1a haftenden Gefrierschnitte wurden 30 Minuten bei Zimmertemperatur in die Tropfen getaucht, danach in $P_H$-gepufferter Kochsalzlösung (Phosphate-buffered saline, PBS) gewaschen, zuerst diskret mit Hilfe eines zweiten Reagenzträgers 7, dann in einem Becherglas. Es folgte für 30 Minuten die zweite Inkubation der Gefrierschnitte mit Fluoreszenz-markiertem Anithumanserum 12. Nach einer weiteren Waschprozedur wurde $P_H$-gepuffertes Glycerin aufgetragen und ein Deckglas darübergelegt. Betrachtung der Gefrierschnitte mit dem Fluoreszenzmikroskop. Bei der Bestimmung der Antikörper gegen Zellkerne in 200 verschiedenen Seren wurden die gleichen Resultate erzielt wie mit der herkömmlichen Methode. Der Arbeitsaufwand war mit der neuen Methode 10 mal und der Reagenzienverbrauch 3 mal geringer als mit der alten.

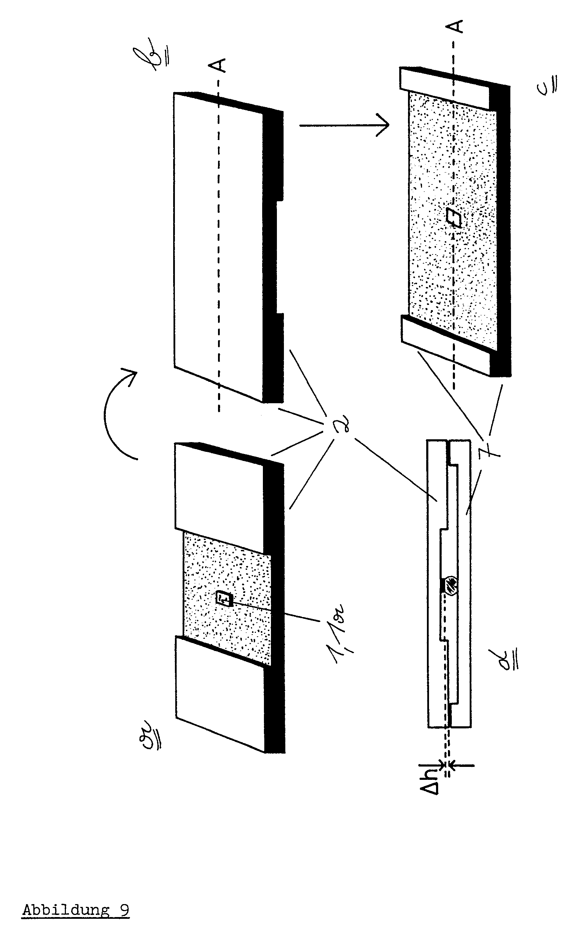

Es können Platten mit beliebig vielen Reaktionsfeldern benutzt werden, z.B. mit einem (Abb. 9) oder mit 48 (Abb. 8).

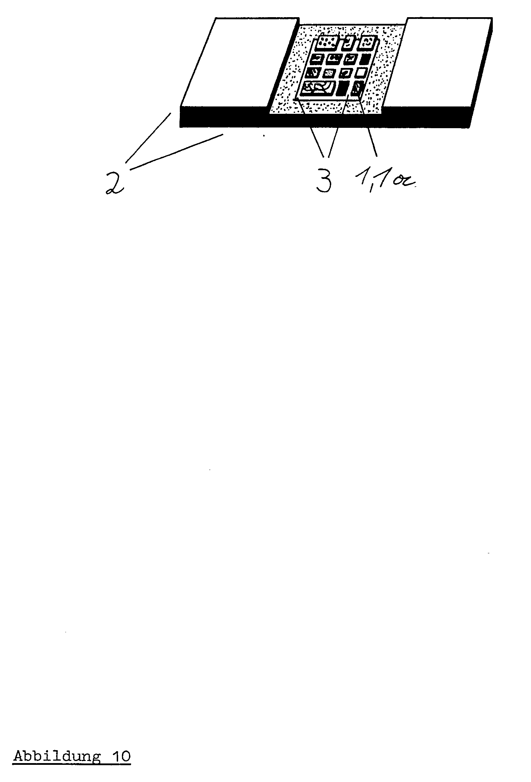

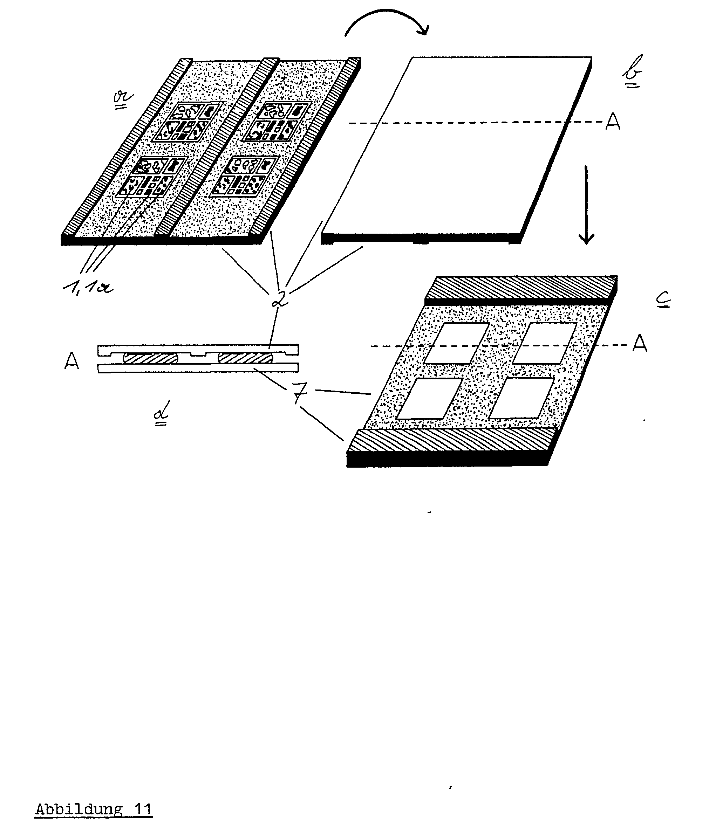

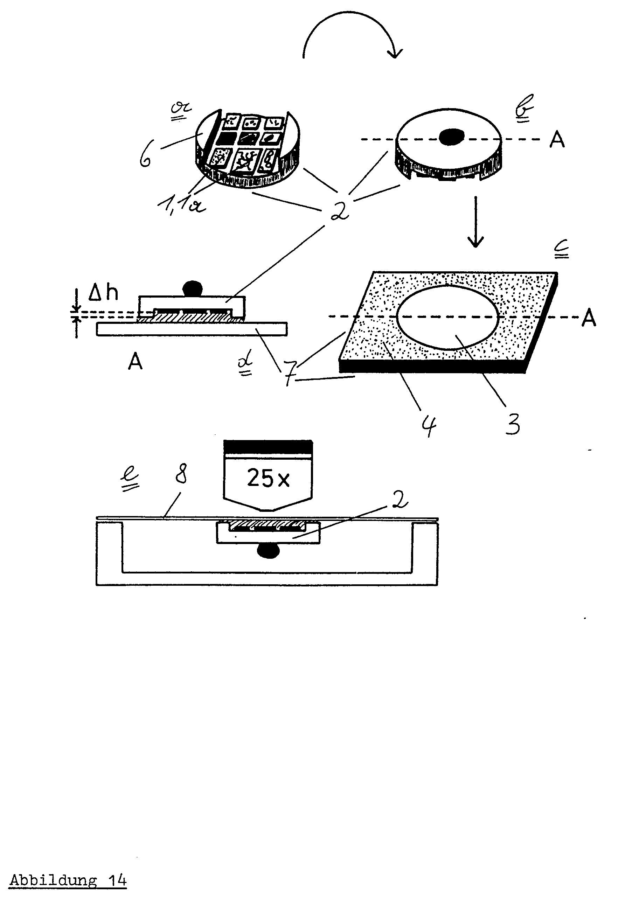

Auf jedes Reaktionsfeld können mehrere Träger mit Gefrierschnitten verschiedener Gewebe geklebt werden («Bunter Schnitt» Abb. 10, 11, 14): Ein Reaktionsfeld pro Platte (Abb. 10, 14), mehrere Reaktionsfelder pro Platte (Abb. 11).

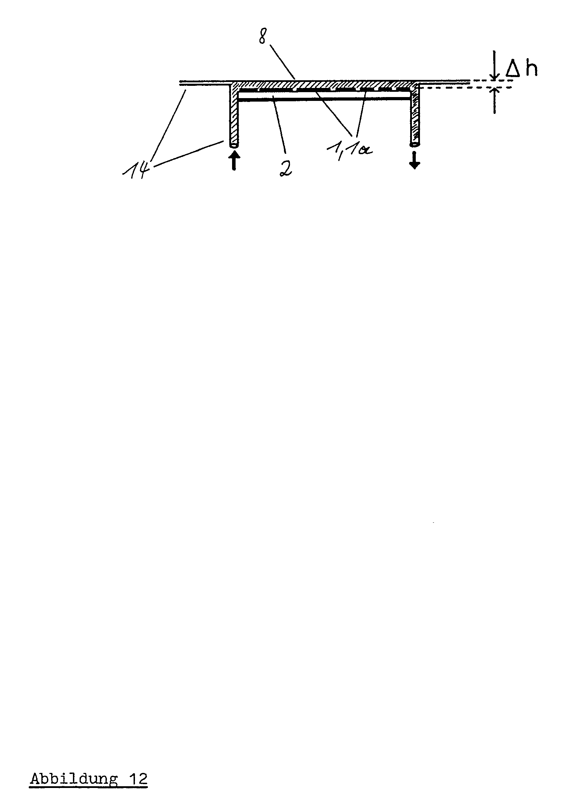

Die Träger mit den Gefrierschnitten können auch auf den Boden einer Durchflussküvette 14 geklebt werden (Abb. 12), als Oberseite dieser Durchflussküvette dient ein Deckglas 8, das in einem sicheren Abstand $\Delta h > 0$ zu den Gefrierschnitten angeordnet ist, aber eine Betrachtung mit dem Mikroskop noch zulässt. Zur Inkubation wird die Durchflussküvette einfach mit der Serum-Verdünnung oder mit dem Fluoreszenz-markierten Antihumanserum gefüllt, zum Waschen wird die $P_H$-gepufferte Kochsalzlösung kontinuierlich durchgepumpt. Die geschlossene Anordnung hat den Vorteil, dass die Gefrierschnitte während des Tests nicht so leicht austrocknen und dass das Laborpersonal weniger durch infektiöse Seren gefährdet ist.

Die Abb. 13 zeigt, dass ein für den Schutz des sich auf der Oberfläche des Trägers 1a befindlichen biologischen Materials notwendiger Abstand $\Delta h$ durch Abstandhalter erzeugt werden kann, die mit einer der Platte 2 gegenüberliegenden Platte (Reagenzträger 7, Deckglas 8) verbunden sind.

In Abb. 14 wird eine scheibchenförmige «Platte» 2 dargestellt, auf die Gefrierschnitt-Fragmente 1a geklebt wurden. Die «Platte» wurde aus einem 0,3 mm starken runden Deckglas gefertigt, auf das zwei 0,3 mm starke Leisten 6 geklebt wurden. Bei den Inkubationen schwimmt die «Platte» auf der Serumverdünnung oder auf dem Fluoreszenz-markierten Antihumanserum, die auf das Reaktionsfeld 3 des Reagenzträgers 7 getropft wurden. Beim Mikroskopieren wird die «Platte» am Deckglas 8 durch Kapillarkraft festgehalten, zwischen die Gefrierschnitte und das Deckglas wurde $P_H$-gepuffertes Glycerin gebracht.

**Patentansprüche**

1. Verfahren für Untersuchungen an unbeweglich gemachtem biologischem Material mit allgemein-biochemischen und mit histochemischen Methoden, insbesondere Methoden der Enzym-, Immun- und Hormonchemie, bei dem eine oder mehrere Untersuchungen nebeneinander auf einer Platte (2) ablaufen und bei dem das biologische Material gegen eine Beschädigung weitgehend geschützt ist, dadurch gekennzeichnet, dass das biologische Material zunächst auf der Oberfläche eines oder mehrerer Träger (1) zur Haftung gebracht wird und danach die Träger oder Teile (1a) der Träger zusammen mit dem biologischen Material auf der Platte (2) befestigt werden, wobei die das biologische Material tragende Oberfläche so angeordnet wird, dass es keinen direkten Kontakt mit festen Gegenständen bekommt und dass es während der Untersuchung nicht austrocknet, wo das unerwünscht ist.

2. Verfahren nach Anspruch 1, bei dem das biologische Material während einer Untersuchung gegen einen direkten Kontakt mit festen Gegenständen geschützt ist, dadurch gekennzeichnet, dass die Träger (1) oder die Teile (1a) der Träger so auf der Platte (2) befestigt werden, dass ihre das biologische Material tragende Oberfläche um den Abstand $\Delta h > 0$ unter der obersten Ebene der Platte (2) oder mit der Platte verbundener Mittel (6) angeordnet ist.

3. Verfahren nach Anspruch 2, bei dem das biologische Material während einer Untersuchung gegen einen direkten Kontakt mit festen Gegenständen und gegen ein Austrocknen weitgehend geschützt ist, dadurch gekennzeichnet, dass die Träger (1) oder die Teile (1a) der Träger auf Reaktionsfeldern (3) der Platte (2) befestigt werden, die rundum von Bereichen der Platte umgeben sind, die die Träger oder die Teile der Träger um den Abstand $\Delta h > 0$ überragen.

4. Verfahren nach Anspruch 1, bei dem das biologische Material während einer Untersuchung gegen einen direkten Kontakt mit festen Gegenständen und gegen ein Austrocknen weitgehend geschützt ist, dadurch gekennzeichnet, dass die Platte (2) als Boden einer Durchflussküvette (14) ausgebildet ist, auf der ein oder mehrere Träger (1) oder Teile (1a) der Träger befestigt sind, dass deren das biologische Material tragende Oberfläche einen Abstand $\Delta h > 0$ zu einer gegenüberliegenden Wand aufweist und dass die für die Untersuchung verwendeten Proben- und Reagenzienlösungen kontinuierlich oder intermittierend durch die Durchflussküvette geleitet werden.

5. Verfahren für histochemische Untersuchungen nach einem der Ansprüche 1 bis 4, dadurch gekennzeichnet, dass als biologisches Material histologische Dünnschnitte biologischen Gewebes verwendet werden.

6. Verfahren nach Anspruch 5, dadurch gekennzeichnet, dass als Träger Glasscheiben benutzt werden.

7. Verfahren nach einem oder beiden der Ansprüche 5 und 6, dadurch gekennzeichnet, dass an die Oberfläche des Trägers (1) vor dem Aufbringen der Gefrierschnitte reaktionsfähige Gruppen gekoppelt werden, die mit den Gefrierschnitten reagieren und sie chemisch binden.

8. Verfahren nach Anspruch 7, dadurch gekennzeichnet, dass als Träger Glas (15) oder ein anderes mit Silanen sich verbindendes Material verwendet wird, dass man an den Träger ein mindestens eine Aminogruppe enthaltendes Silan (16) koppelt und im Anschluss daran an dieses Silan einen Dialdehyd, und dass man darauf den Gefrierschnitt bringt.

9. Vorrichtung zur Durchführung des Verfahrens nach einem oder mehreren der Ansprüche 1–8, gekennzeichnet durch eine Platte (2) mit einem oder mit mehreren Reaktionsfeldern (3) zur Aufnahme der Träger (1) oder der Teile (1a) der Träger, in der die das biologische Material tragende Oberfläche gegen Beschädigung weitgehend geschützt ist, wobei

A. die oberste Ebene der Platte oder die oberste Ebene mit der Platte verbundener Mittel das biologische Material um den Abstand $\Delta h > 0$ überragen, oder

B. die Reaktionsfelder der Platte rundum von Bereichen der Platte umgeben sind, die die Träger oder die Teile der Träger um den Abstand $\Delta h > 0$ überragen oder

C. die Platte als Boden einer Durchflussküvette (14) ausgebildet ist, auf der ein oder mehrere Träger oder Teile der Träger befestigt sind, und deren das biologische Material tragende Oberfläche einen Abstand $\Delta h > 0$ zu einer gegenüberliegenden Wand aufweist, oder

D. durch andere geometrische (13a, 13b) oder durch mechanische oder andere Mittel ein Abstand $\Delta h > 0$ des biologischen Materials zu einer gegenüberliegenden Oberfläche einstellbar ist, und durch Träger (1) oder Teile (1a) der Träger, auf die das biologische Material zur Haftung bringbar ist, und die auf der Platte (2) befestigbar sind,
wobei

a. die Träger aus nicht aktiviertem Glas, Kunststoff oder anderem Material bestehen, oder

b. die Träger aus Glas, Kunststoff oder anderem Material bestehen, und an deren Oberfläche vor dem Aufbringen des biologischen Materials reaktive Gruppen gekoppelt sind, die das biologische Material chemisch binden und

c. Die Oberfläche der Träger nicht mit biologischem Material vorbeschichtet ist, oder

d. die Oberfläche der Träger mit biologischem Material vorbeschichtet ist und

e. auf die Oberfläche der Träger nicht-histologisches biologisches Material aufgebracht ist, oder

f. auf die Oberfläche der Träger histologische Dünnschnitte nicht gefrorenen biologischen Gewebes aufgebracht sind oder

g. auf die Oberfläche der Träger histologische Dünnschnitte gefrorenen biologischen Gewebes aufgebracht sind.

10. Vorrichtung nach Anspruch 9, dadurch gekennzeichnet, dass die Träger (1) oder Teile (1a) der Träger nach der Beschichtung mit dem biologischen Material in Schläuchen, Ampullen oder Ähnlichem eingefroren gelagert sind.

**Claims**

1. Procedure for investigations on immobilized biological material using general biochemical and using histochemical methods, in particular methods of enzyme chemistry, immunochemistry and hormone chemistry, in which one or more investigations in juxtaposition take place on one plate (2) and in which the biological material is substantially protected against damage, characterized in that the biological material is initially induced to adhere to the surface of one or more carriers (1) and then the carriers or parts (1a) of the carriers are, together with the biological material, fixed onto the plate (2), the surface carrying the biological material being arranged such that it does not come into direct contact with solid objects, and such that it does not dry out during the investigations where this is undesired.

2. Procedure according to Claim 1, in which the biological material is protected during an investigation against direct contact with solid objects, characterized in that the carriers (1) or the parts (1a) of the carriers are fixed onto the plate (2) in such a way that their surface carrying the biological material is arranged at the distance $\Delta h > 0$ below the uppermost plane of the plate (2) or means (6) connected to the plate.

3. Procedure according to Claim 2, in which the biological material is substantially protected during an investigation against direct contact with solid objects and against drying out, characterized in that the carriers (1) or the parts (1a) of the carriers are fixed onto reaction fields (3) of the plate (2) which are surrounded all round by regions of the plate which project over the carriers or the parts of the carriers by the distance $\Delta h > 0$.

4. Procedure according to Claim 1, in which the biological material is substantially protected during an investigation against direct contact with solid objects and against drying out, characterized in that the plate (2) is designed as the base of a flow-through cuvette (14) onto which one or more carriers (1) ort parts (1a) of the carriers are fixed, the surface thereof carrying the biological material has a distance $\Delta h > 0$ from an opposite wall, and the sample and reagent solutions used for the investigation are continuously or intermittently passed through the flow-through cuvette.

5. Procedure for histochemical investigations according to one of Claims 1 to 4, characterized in that thin histological sections of biological tissue are used as biological material.

6. Procedure according to Claim 5, characterized in that glass plates are used as carriers.

7. Procedure according to one or both of Claims 5 and 6, characterized in that before the frozen sections are applied to the surface of the carrier 1 there are coupled thereto reactive groups which react with the frozen sections and bond them chemically.

8. Procedure according to Claim 7, characterized in that glass (15) or another material which forms compounds with silanes is used as carrier, a silane (16) containing at least one amino group is coupled to the carrier, and subsequently a dialdehyde is coupled to this silane, and the frozen section is applied thereto.

9. Apparatus for carrying out the procedure according to one or more of Claims 1–8, characterized by a plate (2) having one or having several reaction fields (3) to receive the carriers (1) or the parts (1a) of the carriers, in which the surface carrying the biological material is substantially protected against damage, with

A. the uppermost plane of the plate, or the uppermost plane of the means connected to the plate, projecting over the biological material by the distance $\Delta h > 0$, or

B. the reaction fields of the plate being surrounded all round by regions of the plate which project over the carriers or the parts of the carriers by the distance $\Delta h > 0$ or

C. the plate being designed as the base of a flow-through cuvette (14) onto which one or more carriers or parts of the carriers are fixed, the surface thereof carrying the biological material having a distance $\Delta h > 0$ from an opposite wall, or

D. it being possible to adjust a distance $\Delta h > 0$ of the biological material from an opposite surface by other geometric (13a, 13b) or by mechanical or other means,

and by carriers (1) or parts (1a) of the carriers, onto which the biological material can be induced to adhere, and which can be fixed onto the plate (2), with

a. the carriers consisting of non-activated glass, plastic or other material, or

b. the carriers consisting of glass, plastic or other material, onto the surface of which there have been coupled, before application of the biological material, reactive groups which chemically bond the biological material, and

c. the surface of the carriers not previously having been coated with biological material, or

d. the surface of the carriers having previously been coated with biological material and

e. non-histological biological material having been applied to the surface of the carriers, or

f. thin histological section of non-frozen biological tissue having been applied to the surface of the carriers, or

g. thin histological sections of frozen biological tissue having been applied to the surface of the carriers.

10. Apparatus according to Claim 9, characterized in that the carriers (1) or parts (1a) of the carriers are, after coating with the biological material, stored frozen in tubes, ampoules or the like.

## Revendications

1. Procédé pour faire des analyses employant du matériau biologique immobilisé, par des méthodes d'une manière générale biochimiques et par des méthodes histochimiques, en particulier des méthodes de la chimie enzymathique, immunologique et hormonale, dans lequel un ou plusieurs tests sont effectués les uns après les autres sur une plaque (2) et dans lequel le matériau biologique est largement protégé contre une altération, caractérisé en ce qu'on fait d'abord adhérer le matériau biologique à la surface d'un ou de plusieurs supports (1) et en ce qu'on fixe ensuite les supports ou des partes (1a) de supports ensemble avec le matériau biologique sur la plaque (2), la surface portant le matériau biologique étant agencée de façon que le matériau ne vienne pas en contact direct avec des objets solides et qu'il ne dessèche pas pendant le test, ce qui n'est pas souhaitable.

2. Procédé suivant la revendication 1, dans lequel le matériau biologique est protété contre un contact direct avec des objets solides pendant un test, caractérisé en ce que les supports (1) ou les parties (1a) de supports sont fixés sur la plaque (2) de telle manière que leur surface portant le matériau biologique soit disposée à une distance $\Delta h > 0$ au-dessous du plan supérieur de la plaque (2) ou au-dessous de moyens (6) reliés à la plaque.

3. Procédé suivant la revendication 2, selon lequel le matériau biologique est largement protégé contre un contact direct avec des objets solides et contre un dessèchement pendant un test, caractérisé en ce que les supports (1) ou les parties (1a) de supports sont fixés sur des zones de réaction (3) de la plaque (2), les zones de réaction étant suivant leur pourtour entourées par des zones de la plaque dépassant en hauteur les supports ou les parties de support d'une distance $\Delta h > 0$.

4. Procédé suivant la revendication 1, dans lequel le matériau biologique est largement protégé contre un contact direct avec des objets solides et contre un dessèchement pendant un test, caractérisé en ce que la plaque (2) constitue le fond d'une cuve de circulation (14) sur laquelle un ou plusieurs supports (1) ou parties (1a) de supports sont fixés, en ce que la surface correspondante portant le matériau biologique est disposée à une distance $\Delta h > 0$ par rapport à une paroi opposée, et en ce que les solutions d'échantillons et de réactifs utilisées pour le test sont amenées à travers la cuve de circulation de façon continue ou intermittente.

5. Procédé pour des tests histochimiques suivant l'une des revendications 1 à 4, caractérisé en ce que des coupes minces histologiques de tissu biologique sont utilisées comme matériau biologique.

6. Procédé suivant la revendication 5, caractérisé en ce que des plaques de verre sont utilisées comme support.

7. Procédé suivant la revendication 5 ou 9 ou suivant les deux, caractérisé en ce que des groupes réactifs sont couplés à la surface du support (1) avant la mise en place des coupes congelées, les groupes réactifs réagissant avec les coupes congelées et se liant chimiquement à ceux-ci.

8. Procédé suivant la revendication 7, caractérisé en ce que le verre (15) ou un autre matériau se combinant avec des silanes est utilisé comme support, en ce qu'on couple au support un silane (16) comportant au moins un groupe amino et ensuite un dialdéhyde sur ce silane, en ce qu'on place là dessus la coupe congelée.

9. Dispositif pour la mise en œuvre du procédé suivant une ou plusieurs des revendications 1 à 8, caractérisé par une plaque (2) pourvue d'une ou de plusieurs zones de réaction (3) pour la réception des supports (1) ou des parties (1a) de supports, dans laquelle la surface portant le matériau biologique est largement protégée contre une altération, où

A. le plan supérieur de la plaque ou le plan supérieur de moyens reliés à la plaque dépasse en hauteur le matériau biologique d'une distance $\Delta h > 0$, ou

B. les zones de réaction de la plaque sont sur leur pourtour entourés par des zones de la plaque dépassant en hauteur les supports ou les parties des supports d'une distance Δh > 0, ou

C. la plaque constitue le fond d'une cuve de circulation (14) sur laquelle un ou plusieurs supports ou parties de supports sont fixés et dont la surface portant le matériau biologique est disposée à une distance Δh > 0 par rapport à une paroi opposée, ou

D. par d'autres moyens géométriques (13a, 13b) ou par des moyens mécaniques ou autres une distance Δh > 0 du matériau biologique est réglable par rapport à une surface opposée et des supports (1) ou des parties (1a) de supports sur lesquels le matériau biologique peut être placé pour y adhérer, et qui peuvent être fixés sur la plaque (2), où

a. les supports sont constitués par un verre, une matière synthétique ou un autre matériau non activé, ou

b. les supports sont constitués par un verre, une matière synthétique ou un autre matériau, des groupes réactifs étant couplés aux surfaces des supports avant la mise en place du matériau biologique, les groupes réactifs se liant chimiquement au matériau biologique et

c. la surface des supports n'est pas couverte au préalable d'une couche d'un matériau biologique, ou

d. la surface des supports est couverte au préalable d'une couche d'un matériau biologique et

e. du matériau biologique non histologique est disposé sur la surface des supports, ou

f. des coupes minces histologiques d'un tissu biologique non congelé sont disposées sur la surface des supports ou

g. des coupes histologiques d'un tissu biologique congelé sont disposées sur la surface des supports.

10. Dispositif suivant la revendication 9, caractérisé en ce que les supports (1) ou les parties (1a) de supports sont, après l'application d'une couche du matériau biologique, stockés à l'état congelé dans des tuyaux, des empoules ou similaire.

Objekt (Gefrierschnitt mit
Antigenen)

inkubieren:
mit Antikörperhaltigem Serum

waschen:

inkubieren: mit
Fluoreszenz-
markiertem
Antihumanserum

waschen:

Abbildung 1

Abbildung 2

Abbildung 3

Abbildung 4

17

| G E F R I E R S C H N I T T |

$NH_2$      $N$

**10**

**9** →

Glutardialdehyd

**17**

$HC = O$     $HC$

$(CH_2)_3$     $(CH_2)_3$

$HC$       $HC$

$N$        $N$

$(CH_2)_2$     $(CH_2)_2$

$NH$       $NH$

Aminoäthylaminopropyltrimethoxysilan

**16**

$(CH_2)_3$     $(CH_2)_3$

$-Si-$      $-Si-$

| T R Ä G E R M A T E R I A L    ( G L A S ) |

**15**

Abbildung 5

Abbildung 6

Platte — 2

1, 1α

7 — Reagenzträger

**pipettieren:** je 10 μl
Serum-Verdünnung

11

**inkubieren:** 30 min

**waschen:** 3 x 10 sec
diskret
und 3 x 10 min
im Becherglas
mit PBS

**pipettieren:** je 10 μl
FITC-markiertes
Antihumanglobulin

12

**inkubieren:** 30 min

**waschen:** abspülen und
3 x 10 min im
Becherglas mit PBS

**eindecken:** mit
Glycerin-PBS 9 : 1,
Deckglas;
mikroskopieren

8

25x

Abbildung 7

_a_

_b_

A

_1,1a_

_2_

A

_d_

_7_

_c_

A

Abbildung 8

Abbildung 9

Abbildung 10

Abbildung 11

Abbildung 12

Abbildung 13

Abbildung 14